**Europäisches Patentamt**

**European Patent Office**

**Office européen des brevets**

(19)

(11) Veröffentlichungsnummer: **0 164 560**
**B1**

(12) **EUROPÄISCHE PATENTSCHRIFT**

(45) Veröffentlichungstag der Patentschrift:
**29.10.86**

(51) Int. Cl.⁴: **C 07 C 5/333**, C 07 C 11/09

(21) Anmeldenummer: **85105465.0**

(22) Anmeldetag: **04.05.85**

(54) **Verfahren zum Dehydrieren von Kohlenwasserstoffen.**

(30) Priorität: **05.05.84 DE 3416672**

(43) Veröffentlichungstag der Anmeldung:
**18.12.85 Patentblatt 85/51**

(45) Bekanntmachung des Hinweises auf die Patenterteilung:
**29.10.86 Patentblatt 86/44**

(84) Benannte Vertragsstaaten:
**AT BE CH DE FR GB IT LI LU NL SE**

(56) Entgegenhaltungen:
**DE - A - 2 304 280**
**DE - A - 3 312 515**
**US - A - 3 647 909**
**US - A - 3 978 150**
**US - A - 4 229 603**

(73) Patentinhaber: **VEBA OEL AG,**
**Alexander-von-Humboldt-Strasse,**
**D-4650 Gelsenkirchen-Hassel (DE)**

(72) Erfinder: **Gottlieb, Klaus, Dr., Alte Strasse 59,**
**D-5804 Herdecke (DE)**
Erfinder: **Graf, Wilfried, Lütkenbusch 4, D-4270 Dorsten (DE)**
Erfinder: **Schädlich, Heinz-Kuno, Dr., Am Krausen Bäumchen 129, D-4300 Essen (DE)**

(74) Vertreter: **Krug, Joachim, Dr.,**
**Alexander-von-Humboldt-Strasse,**
**D-4650 Gelsenkirchen-Hassel (DE)**

ACTORUM AG

## Beschreibung

Die vorliegende Erfindung betrifft ein verbessertes Verfahren zur Herstellung ungesättigter Kohlenwasserstoffe durch Dehydrieren von ggf. auch Monoolefine enthaltenden Paraffinkohlenwasserstoffen in Gegenwart eines festen Katalysators. Die katalytische Dehydrierung von Kohlenwasserstoffen, insbesondere von kurzkettigen Kohlenwasserstoffen mit zwei bis sechs Kohlenstoffatomen, erfolgt in bekannter Weise dadurch, dass die Kohlenwasserstoffe, rein oder mit Inertgas verdünnt, adiabatisch durch Festbettreaktoren, die zu mehreren parallel geschaltet sein können, geleitet werden. Die Dehydrierungskatalysatoren bestehen im allgemeinen aus Aluminiumoxid und Zusätzen, wie z.B. Chromoxid oder Platin, die z.B. durch Tränken mit wässerigen Lösungen entsprechender Salze oder durch Ausfällen auf Aluminiumoxid aufgebracht werden.

Die durch Dehydrieren von paraffinischen Kohlenwasserstoffen erzeugten Olefine, insbesondere kurzkettigen Olefine werden z.B. bei der Alkylierung von Kohlenwasserstoffen oder bei der Verätherung von Alkoholen eingesetzt. Die daraus gewonnenen Endprodukte — Alkylat bzw. Äther — sind wichtige Zusätze zur Herstellung klopffester Vergaserkraftstoffe. Das bei der Dehydrierung von Buten erhaltene Butadien kann z.B. zur Herstellung von künstlichem Kautschuk verwendet werden.

Während der Dehydrierungsreaktion lagert sich Koks auf dem festen Katalysator ab, wodurch die für die Reaktion benötigten aktiven Zentren blockiert werden. Zur Wiederherstellung der Katalysatoraktivität wird der Koks in bekannter Weise in einem sauerstoffhaltigen Gas, z.B. einem Gemisch von Luft und Rauchgasen oder Stickstoff, abgebrannt. Hieraus ergibt sich eine zyklische Arbeitsweise, bei der Dehydrieren der Kohlenwasserstoffe und Regeneration des Katalysators, jeweils getrennt durch Spülen mit Inertgasen oder Evakuieren, einander abwechseln. Entsprechende Verfahren sind z.B. in den US-PSen 3,647,909, 3,711,569, 3,781,376, 4,012,335 und 4,371,730 beschrieben.

Die Kohlenwasserstoffe werden bei Temperaturen von 500 bis 650° C mit dem Katalysator in Kontakt gebracht. Nach einem relativ kurzen Zeitraum, der, je nach den Reaktionsbedingungen, einige Minuten bis zu einer Stunde dauern kann, geht der Kohlenwasserstoffumsatz merklich zurück. Dies beruht zum einen auf der bereits erwähnten Verminderung der Katalysatoraktivität durch den auf den Katalysator abgelagerten Koks, zum anderen auf der Kühlung des Katalysatorbettes durch die Reaktion, da die Wärmemenge, die dem Katalysatorbett infolge der endothermen Reaktion entzogen wird, grösser ist als die, die mit dem Kohlenwasserstoffstrom zugeführt werden kann.

Die durch die Oxidation des Kokses während der Regenerierphase entstehende Wärmemenge reicht bei guter Reaktionsführung, d.h. geringer Koksabscheidung während der Dehydrierung nur aus, einen Teil der durch die endotherme Dehydrierreaktion verbrauchten Wärmemenge zu ersetzen und das Katalysatorfestbett auf die für eine adiabatische Durchführung der Dehydrierreaktion notwendigen Temperatur aufzuheizen. Daher wird das für die Regenierung verwendete sauerstoffhaltige Gas auf Temperaturen von 600 bis 700° C vorgeheizt und während der Regenerierphase länger, als zur Oxidation des Kokses notwendig, durch das Katalysatorbett geleitet.

Eine in den genannten US-PSen beschriebene und in vielen technischen Anlagen geübte Verfahrensweise ist es auch, einen Teil der zum Wiederaufheizen des Katalysatorenbettes notwendigen Wärme zuzuführen, dass dem sauerstoffhaltigen Gas vor Eintritt in den Reaktor eine der gewünschten Wärmemenge äquivalente Menge eines gasförmigen oder flüssigen Brennstoffes (injection fuel) zugesetzt wird, die dann im Katalysatorraum verbrennt und dabei die gewünschte Wärmemenge erzeugt.

Bei den bekannten Verfahren durchströmt das sauerstoffhaltige Gas den Reaktor während der Regenerationsphase in der gleichen Richtung wie das eingesetzte Kohlenwasserstoffgemisch während der Dehydrierphase, d.h Kohlenwasserstoffe und Regenerationsgas treten zeitlich versetzt an der gleichen Stelle in das Katalysatorbett ein. Beim Durchgang durch das Katalysatorbett kühlt das Regenerationsgas ab. Da für die Regenerierphase nur eine beschränkte Zeit zur Verfügung steht, kann in der Schüttschicht kein stationärer Zustand erreicht werden. Im Katalysatorbett bildet sich daher ein mehr oder weniger steiles Temperaturgefälle zum Reaktorenende hin aus.

Da mit sinkender Temperatur der Umsatz der Kohlenwasserstoffe abnimmt, bringt dieses fallende Temperaturprofil während der Dehydrierphase einen Verlust an Reaktionsprodukt. Dies ist vor allem deshalb von grossem Nachteil, weil gerade zu Beginn der Reaktionsphase die Aktivität des frisch regenerierten Katalysators sehr gross ist und eine niedrige Temperatur sich somit besonders stark auf die Ausbeute auswirkt.

Um die Temperatur im Reaktor während der Dehydrierphase zu verringern, werden die Kohlenwasserstoffe vorerhitzt in das Katalysatorbett geleitet. Je nach Höhe der Vorerhitzung und der damit verbundenen Verringerung des Temperaturabfalles treten jedoch Crackreaktionen auf, die zu merklichen Ausbeuteverlusten führen.

Die vorliegende Erfindung hat sich die Aufgabe gestellt, die Nachteile der bisherigen Arbeitsweise zu vermeiden und den Energieeinsatz bei der Dehydrierung von Kohlenwasserstoffen zu verringern, wodurch die Wirtschaftlichkeit des Verfahrens erheblich verbessert wird. Gleichzeitig soll das Spektrum der beim Verfahren erhaltenen Produkte so verändert werden, dass die Kosten der der Dehydrierstufe folgenden Aufarbeitungsstufen bedeutend gesenkt werden. Erfindungsgemäss wird während der Regenerationsphase im Katalysatorfestbett ein mit der Strömungsrichtung der zu dehydrierenden Kohlenwasserstoffe ansteigendes Temperaturprofil erzeugt. Erreicht wird dies dadurch, dass im Gegensatz zu den bisher bekannten

Verfahren das zur Regeneration des Katalysators verwandte sauerstoffhaltige Gas, dem ggf. ein gasförmiger oder flüssiger Brennstoff zugesetzt wurde (injection fuel), das Katalysatorbett in einer Richtung durchströmt, die der der Kohlenwasserstoffe in der Dehydrierphase entgegengesetzt ist.

Wegen der Abkühlung des Regeneriergases entgegengesetzt zur Strömungsrichtung der Kohlenwasserstoffe stellt sich am Kohlenwasserstoffeintritt in die Katalysatorschicht eine niedrigere Temperatur ein als am Kohlenwasserstoffaustritt aus der Katalysatorschicht. Die Temperatur des Katalysator-Festbettes kann durch Menge und Temperatur des Regeneriergasgemisches sowie des Sauerstoffgehaltes und der Menge des ggf. zugesetzten gasförmigen oder flüssigen Brennstoffes (injection fuel) so geregelt werden, dass das frisch eintretende Reaktionsgas eine für den Umsatz optimale Temperatur vorfindet. Während das Kohlenwasserstoffgemisch das Festbett durchströmt, gleichen sich die ansteigende Katalysatortemperatur und die mit der fortschreitenden Reaktion fallende Temperatur des Reaktionsgases so an, dass im Zeitmittel die Reaktion bei einer Temperatur abläuft, die Umsatz und Selektivität auf einem hohen Niveau hält.

Das erfindungsgemässe Verfahren hat den weiteren Vorteil, die unerwünschten Crackreaktionen zurückzudrängen. Da zur Aufrechterhaltung der Reaktionstemperatur die Kohlenwasserstoffe vor Eintritt in den Reaktor weniger hoch erhitzt werden müssen als beim bekannten Verfahren, ist ihre Neigung zu Crackreaktionen geringer. Hierdurch wird auch die Selektivität des Verfahrens für das gewünschte olefinische Reaktionsprodukt erhöht.

Beim erfindungsgemässen Verfahren gestaltet sich die Abtrennung und Reinigung der Reaktionsprodukte einfacher als beim früheren Verfahren. Durch Anwendung der erfindungsgemässen Gegenstrom-Regeneration werden grössere Mengen Einsatzprodukte bei gleicher Selektivität dehydriert als beim bekannten Verfahren mit Gleichstrom-Regeneration. Somit enthält das die Reaktoren verlassende Dehydrierungsprodukt geringere Mengen an nicht umgesetzten Kohlenwasserstoffen und an Crackgasen. Die Abtrennung der gewünschten Olefine wird hierdurch erleichtert und ihre Produktion verbilligt.

Aus DE-OS 23 04 280 ist es bekannt, bei der Regenerierung eines Bauxit-Katalysators, der zur Herstellung von elementarem Schwefel durch Teilverbrennung eines Schwefelwasserstoffstromes mit Luft verwendet wurde und auf dem durch Kohlenwasserstoffe, die in den Ausgangsstoffen enthalten waren, koksartige Ablagerungen gebildet wurden, diese mit molekularem Sauerstoff in Gegenwart eines Inertgases abzubrennen, wobei das Regenerationsgas in einer zum normalen Betrieb der Schwefelrückgewinnungsanlage entgegengesetzten Strömungsrichtung geleitet wird. Dieser Gegenstrombetrieb wurde dort deshalb gewählt, weil erhebliche Mengen von flüssigem Schwefel, die in der Konversionszone zurückbleiben und vom Spülgas nicht ausgetrieben werden, durch die physikalische Einwirkung des Regeneriermittelstromes beseitigt werden können. Da bei den katalytischen Dehydrierverfahren andere Bedingungen als beim Schwefelrückgewinnungsverfahren vorliegen und Zweck und Effekt der Führung des Regenerationsgasstromes in entgegengesetzter Richtung zur Führung des Reaktionsgasstromes beim Schwefelrückgewinnungsverfahren anders sind als beim Dehydrierverfahren, konnte die beim Schwefelrückgewinnungsverfahren bekannte Massnahme ihre Anwendung beim Dehydrierverfahren nicht nahelegen.

Unter Bezugnahme auf die zugehörige Zeichnung wird das erfindungsgemässe Verfahren erläutert. Mit A, B und C sind drei Festbett-Reaktoren bezeichnet, in denen sich die eingangs erwähnten Dehydrierungskatalysatoren, insbesondere Chromoxid oder Platin auf Aluminiumoxid befinden. Jeder der Reaktoren ist über ein Ventil 4A, 4B und 4C an die Kohlenwasserstoff-Zuführung 10 und über ein Ventil 7A, 7B und 7C an die Abführungsleitung 12 für das dehydrierte Produkt angeschlossen. Das zur Regeneration der Katalysatoren verwendete sauerstoffhaltige Gas wird über die Zufuhr 14 und die Ventile 6A, 6B und 6C den Reaktoren zugeführt und über Ventile 1A, 1B und 1C durch Leitung 13 abgeführt. Falls erforderlich kann mittels Leitung 15 sowie Ventilen 5A, 5B und 5C gasförmiger oder flüssiger Brennstoff zur Erzeugung der gewünschten Wärmemenge während der Regeneration zugeführt werden. Zur Evakuierung der Reaktoren zwischen Regenerierung und Dehydrierung dient Leitung 11 in Verbindung mit den Ventilen 2A, 2B und 2C. Alternativ kann die Evakuierung auch über Leitung 9 in Verbindung mit den Ventilen 8A, 8B und 8C erfolgen. Durch Leitung 16 und die Ventile 3A, 3B und 3C schliesslich kann den Reaktoren ein reduzierendes Gas, z.B. Wasserstoff zugeführt werden, wenn dies zur Wiederherstellung der Katalysatoraktivität notwendig ist.

Der Ablauf eines Reaktions/Regenerations-Zyklus wird am Beispiel des Reaktors A beschrieben. Vor der Reaktionsphase wird der Reaktor evakuiert. Hierzu wird das Ventil 2A geöffnet, die übrigen Ventile 1A, 3A bis 7A sind geschlossen.

Für die Dehydrierungsphase wird Ventil 2A geschlossen, während Ventile 4A und 7A geöffnet werden. Der Einsatzstrom der Kohlenwasserstoffe fliesst durch Leitung 10 in den Reaktor; die Reaktionsprodukte verlassen den Reaktor durch Leitung 12.

Als Kohlenwasserstoff-Einsatzprodukt kann beim erfindungsgemässen Verfahren jedes Alkan oder Monoolefin verwendet werden. Kurzkettige Kohlenwasserstoffe mit 2 bis 6 Kohlenstoffatomen werden bevorzugt. Zum Beispiel kann das Einsatzprodukt Propan, n-Butan, n-Pentan, i-Butane und i-Pentane sowie Mischungen von zwei oder mehr dieser gesättigten Kohlenwasserstoffe enthalten. Insbesondere soll das Verfahren bei der Dehydrierung von Kohlenwasserstoffgemischen verwendet werden, deren Hauptbestandteil Isobutan ist. Darüber hinaus kann es auch merkliche Anteile olefinischer Nebenprodukte wie Propen, Butadien, Pentadien und die verschiedenen Butene und

Pentene enthalten. Die olefinischen Nebenprodukte können z.B. durch Rückführen von nicht umgesetztem Alkan oder Monoolefin in das Kohlenwasserstoff-Einsatzprodukt gelangen.

Die Kohlenwasserstoffe werden rein oder mit einem Inertgas verdünnt adiabatisch durch den Festbett-Reaktor geleitet. Dabei wird dieser mit 0,1 bis 5 Gewichtsteilen Einsatzprodukt pro Gewichtsteil Katalysator und Stunde beaufschlagt. Das Einsatzprodukt ist auf 500 bis 680° C vor Eintritt in den Katalysator vorgeheizt und erwärmt sich beim Eintritt in den Katalysator auf Temperaturen zwischen 540 und 700° C. Der Umsatz, d.h. der dehydrierte Anteil des Einsatzproduktes liegt zwischen 10 und 85 Gew.-%, insbesondere zwischen 40 und 70 Gew.-%. Die Selektivität für das Produkt beträgt 90 bis 98 Mol-%. Im bevorzugten Umsetzungsbereich liegt die Selektivität zwischen 85 und 96 Mol-%; die Ausbeuten erreichen 10 bis 70 Mol-%, insbesondere 35 bis 66 Mol-%.

Aus Sicherheitsgründen ist ein Spülen nach der Dehydrierphase vor Beginn der Regeneration notwendig. Das Spülmittel, z.B. Wasserdampf kann über die Einsatzproduktleitung 10 zugeführt und über die Produktleitung 12 abgezogen werden. Zusätzliche Ventile, die in der schematischen Zeichnung nicht dargestellt sind, ermöglichen die Trennung von Spülmittel und Reaktionsprudukt.

Nach Schliessen der Ventile 4A und 7A wird durch die Leitung 14 und das Ventil 6A sauerstoffhaltiges Regenerationsgas in den Reaktor geführt. Als Regenerationsmittel dienen Mischungen von Luft mit Rauchgasen oder mit Stickstoff, deren Sauerstoffgehalt zwischen 2 und 20 Gew.-% liegt. Neben 50 bis 98 Gew.-% Stickstoff kann das sauerstoffhaltige Gas auch bis zu 18% Wasser, bis zu 30% Kohlendioxid und weniger als 1% der in Rauchgasen vorhandenen Verunreinigungen wie Kohlenmonoxid, Schwefeldioxid und Stickoxide enthalten.

Das bei der Regeneration des Katalysatorbettes nicht verbrauchte sauerstoffhaltige Gas verlässt zusammen mit den Verbrennungsprodukten des auf dem Katalysator abgeschiedenen Kohlenstoffs den Reaktor durch das inzwischen geöffnete Ventil 1A und Leitung 13.

Die Temperatur des Festbettkatalysators kann durch Menge und Temperatur des Regeneriermittels beeinflusst werden. Auch durch die Zusammensetzung des sauerstoffhaltigen Gases, d.h. seinen Sauerstoffgehalt sowie ggf. die Menge an zusätzlich zugeführtem Brennstoff kann die Reaktortemperatur gesteuert werden.

Zur Erhöhung der Temperatur im Reaktor kann es zweckmässig sein, einen gasförmigen oder flüssigen Brennstoff mit dem sauerstoffhaltigen Gas dem Reaktor zuzuführen. Dies geschieht über Leitung 15 und Öffnen des Ventiles 5A. Hierdurch ist es möglich, zusätzliche Wärmemengen in das Katalysatorbett einzubringen.

Da die Abkühlung des sauerstoffhaltigen Regenerationsgases in dessen Fliessrichtung erfolgt, also entgegengesetzt zur Fliessrichtung der Kohlenwasserstoffe während der Dehydrierung, stellt sich am Eingangsort der Kohlenwasserstoffbeschickung in den Reaktor eine geringere Temperatur im Katalysatorbett ein als an dem Ende des Katalysatorbettes, an dem das bei der Dehydrierung entstandene Stoffgemisch die Katalysatorpackung verlässt. So ergibt sich ein Temperatur-Abfall im Katalysatorbett zwischen 0 und 160° C.

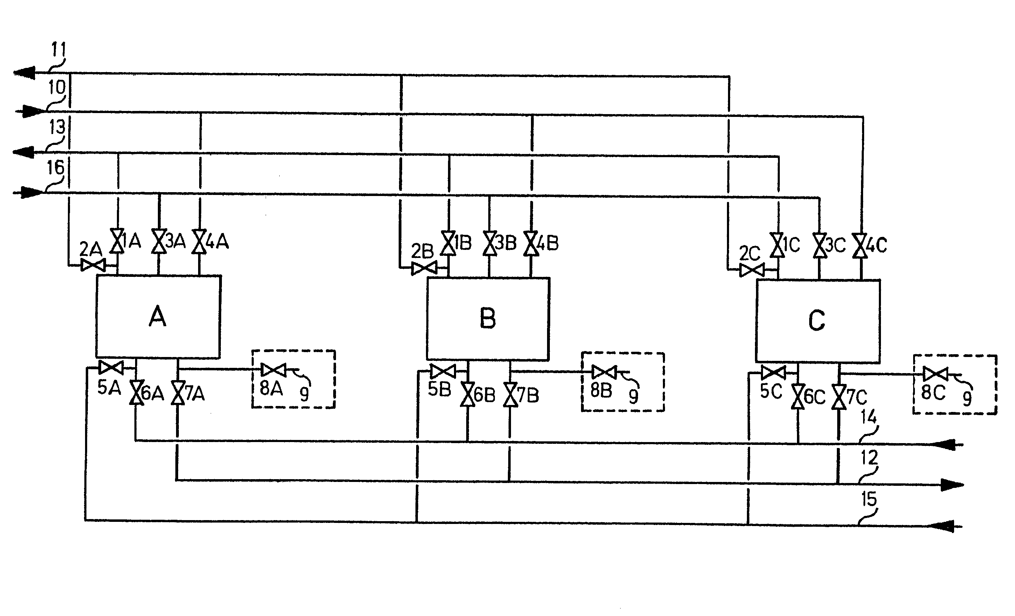

Das frisch zugeführte Kohlenwasserstoff-Einsatzprodukt findet somit im Katalysatorbett die optimale Temperatur für die Reaktion vor. Mit Durchfluss der Kohlenwasserstoffe durch das Festbett kompensieren sich die ansteigende Katalysatortemperatur und die infolge der Reaktion abfallende Temperatur des Kohlenwasserstoff-Einsatzproduktes. Im Zeitmittel verläuft so die Reaktion bei einer Temperatur ab, die Umsatz und Selektivität begünstigt. Nach Beendigung der Regenerierung werden die Ventile 1A und 6A, ggf. auch 5A geschlossen und der Reaktor in der beschriebenen Weise durch Öffnen des Ventils 2A über Leitung 11 evakuiert. Anschliessend kann, nach Schliessen des Ventils 2A und Öffnen des Ventiles 3A, der Katalysator mit Wasserstoff behandelt werden, der über Leitung 16 zugeführt wird. Die Abführung erfolgt über das hierzu geöffnete Ventil 7A und Leitung 12 sowie über weitere, in der Zeichnung nicht wiedergegebene Ventile aus der Produktleitung 12.

In gleicher Weise, wie für den Reaktor A beschrieben, wechseln sich bei den Reaktoren B und C Dehydrier- und Regenerierphasen ab, so dass sich jeweils mindestens ein Reaktor in der Dehydrierphase befindet. Trotz der diskontinuierlichen Arbeitsweise der einzelnen Reaktoren erhält man so einen kontinuierlichen Fluss des Reaktionsproduktes. Wenn es gelingt, das Spülen, Regenerieren und Evakuieren eines Reaktors in der gleichen Zeit auszuführen, in der die Dehydrierphase abläuft, kann eine kontinuierliche Arbeitsweise auch mit zwei Reaktoren erreicht werden. Andernfalls muss, wie beim Arbeiten mit einem Reaktor allein, durch Installation entsprechender Pufferbehälter ein kontinuierliches Arbeiten der vor- und nachgeschalteten Anlagen ermöglicht werden.

Im nachfolgenden Beispiel wird der Fortschritt des erfindungsgemässen Verfahrens gegenüber dem bekannten am Umsatz, der Ausbeute und Selektivität gezeigt.

### Beispiel

Ein Rohrreaktor von 40 mm Durchmesser und 300 mm Länge wurde mit einem Chromoxid-Katalysator befüllt und entsprechend der beschriebenen Vorgehensweise abwechselnd mit einem Kohlenwasserstoffgemisch und einem sauerstoffhaltigen Gas nachstehender Zusammensetzung beschickt.

### Kohlenwasserstoffgemisch

1,08 Gew.-% Propan
98,11 Gew.-% Isobutan
0,40 Gew.-% n-Butan
0,13 Gew.-% Buten-I
0,28 Gew.-% Isobuten

*Sauerstoffhaltiges Gas*

16 Gew.-% Sauerstoff
84 Gew.-% Stickstoff

Der Katalysator wurde durch Tränken von Aluminiumoxid mit Chromnitrat hergestellt. Nach Trocknung und Calcinierung enthielt der Katalysator 17,4 Gew.-% Chromoxid. Als Aluminiumoxid wurde handelsübliches $\gamma$-$Al_2O_3$ der Firma Harshaw verwendet.

Der Reaktor wurde so betrieben, dass eine Reaktionsphase (Dehydrieren) von 9 Minuten und eine Regenerationsphase von insgesamt 18 Minuten einander abwechselten. In der Regenerationsphase wurde vor dem Abbrennen des Kokes 2 Minuten lang mit Stickstoff gespült und nach dem Abbrennen des Kokses 2 Minuten lang evakuiert. Der Reaktor wurde mit 0,5 Gewichtsteilen des genannten Kohlenwasserstoffgemisches pro Stunde und pro Gewichtsteil Katalysator beschickt. Das Kohlenwasserstoffgemisch wurde mit Hilfe eines Vorheizers auf eine Temperatur von 630° C am Eintritt in die Katalysatorschüttschicht erhitzt. In der Katalysatorschüttschicht wurde während der Regenerationsphase ein Temperaturgefälle von 60° C Temperaturdifferenz dadurch eingestellt, dass das auf 640° C erhitzte sauerstoffhaltige Gas den Reaktor entgegengesetzt zur Fliessrichtung der Kohlenwasserstoffe durchströmte, wobei sich am Eintritt der Kohlenwasserstoffe in die Katalysatorschüttschicht eine Temperatur von 580° C und am Austritt der Kohlenwasserstoffe eine Temperatur von 640° C einstellte.

Die den Reaktor verlassenden Produkte, Kohlenwasserstoffe während der Reaktionsphase bzw. $CO_2$haltiges Abbrenngas während der Regeneration, wurden gesammelt, gaschromatographisch analysiert und hieraus der Gesamtumsatz für Isobuten und die Selektivität der Umwandlung zu Isobuten ermittelt.

Unter den vorgenannten Bedingungen betrug der Umsatz an Isobutan 66,8 Mol-%, die Selektivität für Isobuten 91,3 Mol.-%, die Ausbeute an Isobuten somit 61 Mol-%.

*Vergleichsbeispiel*

Das in vorstehendem Beispiel geschilderte Verfahren wurde mit dem alleinigen Unterschied wiederholt, dass das auf 640° C erhitzte sauerstoffhaltige Gas und das auf 630° C erhitzte Kohlenwasserstoffgemisch den Reaktor in derselben Fliessrichtung durchströmte, wodurch sich das Temperaturgefälle so einstellte, dass die Temperatur am Eintritt der Kohlenwasserstoffe in die Katalysatorschüttschicht 580° C betrug.

Unter diesen Bedingungen war der Umsatz an Isobutan nur 60,3 Mol-%, die Selektivität für Isobuten 91,8 Mol-%, die Ausbeute an Isobuten somit nur 55,4 Mol-%.

**Patentansprüche**

1. Verfahren zum Dehydrieren von Kohlenwasserstoffen, bei dem die Kohlenwasserstoffe durch ein Katalysator-Festbett geleitet werden und der Katalysator diskontinuierlich mittels durchgeleiteter sauerstoffhaltiger Gase regeneriert wird, dadurch gekennzeichnet, dass die Strömungsrichtung der sauerstoffhaltigen Gase während der Regenerierphase der Strömungsrichtung der Kohlenwasserstoffe während der Dehydrierungsphase entgegengesetzt ist.

2. Verfahren nach Anspruch 1, dadurch gekennzeichnet, dass die Kohlenwasserstoffe adiabatisch durch das Katalysator-Festbett geleitet werden.

3. Verfahren nach Anspruch 1 oder 2, dadurch gekennzeichnet, dass die Kohlenwasserstoffe zwei bis sechs Kohlenstoffatome aufweisen.

4. Verfahren nach Anspruch 3, dadurch gekennzeichnet, dass die Kohlenwasserstoffe Isobutan als Hauptbestandteil enthalten.

5. Verfahren nach einem der Ansprüche 1 bis 4, dadurch gekennzeichnet, dass die Kohlenwasserstoffe vor der Leitung über das Katalysator-Festbett auf Temperaturen zwischen 500° C und 680° C erhitzt werden.

**Revendications**

1. Procédé pour déshydrogéner des hydrocarbures, procédé dans lequel les hydrocarbures passent à travers un lit solide de catalyseur, tandis que ce catalyseur est régénéré de façon discontinue par des gaz contenant de l'oxygène, qui le traversent, procédé caractérisé en ce que le sens d'écoulement, pendant la phase de régénération des gaz contenant de l'oxygène, est inversé par rapport à celui des hydrocarbures pendant la phase de déshydrogénation.

2. Procédé selon la revendication 1, caractérisé en ce que les hydrocarbures circulent adiabatiquement à travers le lit solide de catalyseur.

3. Procédé selon la revendication 1 ou 2, caractérisé en ce que les hydrocarbures comportent deux à six atomes de carbone.

4. Procédé selon la revendication 3, caractérisé en ce que les hydrocarbures contiennent, en tant que partie constituante principale, de l'isobutane.

5. Procédé selon une des revendications 1 à 4, caractérisé en ce que les hydrocarbures, avant de passer sur le lit solide de catalyseur, sont chauffés à des températures comprises entre 500 et 680° C.

**Claims**

1. Process for the dehydrogenation of hydrocarbons, wherein the hydrocarbons are passed through a fixed bed of catalyst, and the catalyst is regenerated discontinuously by means of oxygen-containing gases passed through said bed, characterized in that during the regeneration phase the flow direction of the oxygen-containing gases is the opposite of the flow direction of the hydrocarbons during the dehydrogenation phase.

2. Process according to claim 1, characterized in that the hydrocarbons are passed adiabatically through the fixed bed of catalyst.

3. Process according to claim 1 or 2, characterized in that the hydrocarbons have two to six atoms of carbon.

4. Process according to claim 3, characterized in that the hydrocarbons contain isobutane as main component.

5. Process according to one of the claims 1 to 4, characterized in that the hydrocarbons are heated to temperatures between 500° C and 680° C before passing through the fixed bed of catalyst.